# EUROPÄISCHE PATENTSCHRIFT

(11) **EP 1 828 361 B1**
(45) Veröffentlichungstag und Bekanntmachung des Hinweises auf die Patenterteilung: **05.03.2014**
(21) Anmeldenummer: 05820568.3
(22) Anmeldetag: 20.12.2005
(51) Int. Cl.: A23K 1/14, A23K 1/16, A23D 9/00

(54) **VERFAHREN ZUR HERSTELLUNG EINES ROHÖLS AUS GEMISCHEN VON MIKROORGANISMEN UND PFLANZEN, DAS SO HERGESTELLTE ÖL SOWIE DIE SPEZIFISCHEN VERWENDUNGEN DES SO HERGESTELLTEN UND GEGEBENENFALLS ZUSÄTZLICH RAFFINIERTEN ÖLS**
METHOD FOR PRODUCING A RAW OIL FROM MIXTURES OF MICRO-ORGANISMS AND PLANTS, OIL PRODUCED ACCORDING TO SAID METHOD AND SPECIFIC USES OF THE THUS PRODUCED OIL AND, OPTIONALLY, ADDITIONAL REFINED OIL
PROCEDE DE PRODUCTION D'UNE HUILE BRUTE A PARTIR DE MELANGES DE MICRO-ORGANISMES ET DE PLANTES, HUILE PRODUITE PAR CE PROCEDE ET UTILISATIONS SPECIFIQUES DE L'HUILE PRODUITE PAR CE PROCEDE ET EVENTUELLEMENT RAFFINEE

(30) Priorität: 23.12.2004 DE 102004062141
(43) Veröffentlichungstag der Anmeldung: 05.09.2007
(73) Patentinhaber: Lonza Ltd, 4002 Basel (CH)
(72) Erfinder: FABRITIUS, Dirk, 91522 Ansbach (DE)
(74) Vertreter: Mai, Dörr, Besier
(86) Internationale Anmeldenummer: PCT/EP2005/013688
(87) Internationale Veröffentlichungsnummer: WO 2006/069668

(56) Entgegenhaltungen:
- EP-A- 0 743 355
- WO-A-97/36996
- WO-A-2004/022678
- WO-A1-02/36996
- WO-A1-92/12711
- WO-A1-02/072742
- US-B1- 6 255 505
- DATABASE FSTA [Online] INTERNATIONAL FOOD INFORMATION SERVICE (IFIS), FRANKFURT-MAIN, DE TAGUCHI K ET AL: 'Changes in dietary fiber of natto and tempeh during fermentation. (translated)' Database accession no. FS-1987-08-J-0115 & Bd. 39, Nr. 3, JOURNAL OF JAPANESE SOCIETY OF NUTRITION AND FOOD SCIENCE [NIHON EIYO SHOKURYO GAKKAI-SHI] 1986 DEP. OF FOOD SCI., KYOTO PREFECTURAL UNIV., KYOTO. 606, JAPAN, Seite 203

## Beschreibung

Verfahren zur Herstellung eines Rohöls aus Gemischen von Mikroorganismen und Pflanzen.

Die vorliegende Erfindung betrifft ein Verfahren zur Herstellung eines Rohöls aus Gemischen von Mikroorganismen und Pflanzen.

Öle, welche langkettige mehrfach ungesättigte Fettsäuren wie beispielsweise Arachidonsäure (ARA), Docosapentaensäure (DPA), Docosahexaensäure (DHA), Eicosapentaensäure (EPA), Stearidonsäure (SA) oder Dihomogammalinolensäure (DHGLA) enthalten, können aus der Kultivierung von Mikroorganismen gewonnen werden. Hierzu wird normalerweise ein Mikroorganismus, der reich an einer oder mehrerer der genannten Fettsäuren ist, gezüchtet, die Biomasse wird aus der Kultur geerntet, aufgeschlossen und das Öl isoliert. Zur Isolierung des Öls aus der Biomasse wurden bisher vor allem Extraktionsverfahren mit organischen Lösungsmitteln, beispielsweise Hexan, oder mit superkritischen Flüssigkeiten verwendet. Im Allgemeinen wird das Öl aus der Biomasse durch Perkolation der getrockneten Biomasse mit Hexan extrahiert. Solche Extraktionen mit organischen Lösungsmitteln sind beispielsweise in der WO97/37032, der WO97/43362, der EP515460 und vielen anderen Dokumenten mehr beschrieben. Eine besonders ausführliche Darstellung findet sich auch im Journal of Dispersion Science and Technology, 10, 561-579, 1989 "Biotechnological Processes for the Production of PUFAs".

Es wird normalerweise angestrebt, einen möglichst hohen Triglyceridanteil im fertigen Öl zu erreichen, insbesondere wenn es für Speisemittel Verwendung finden oder pharmazeutisch/kosmetischen Zwecken zugeführt werden soll. Phospholipide und freie Fettsäuren gelten als Verunreinigungen im fertigen Öl. Lösungsmittel sollen im fertigen Öl ebenfalls nicht mehr in nennenswerten Anteilen vorhanden sein. Bei Verwendung einer Biomasse mit hohem Phospholipidanteil hat, kann man die Phospholipide gegebenenfalls ohne Zusatz weiterer Lösungsmittel (z.B. Ethanol) mithilfe der natürlich vorhandenen Triglyceride, die ja auch als Lösungsmittel wirken, extrahieren. Insbesondere Phospholipide enthaltend PUFAs sind schwer zu isolieren, teuer, aber für viele Anwendungen wünschenswert.

Daher bietet die Extraktion der PUFAs mit Lösungsmitteln eine Reihe prinzipieller Nachteile. So können die PUFAs während der Extraktion mit heißen Lösungsmitteln oder der Abdestillierung des Lösungsmittels mit Luftsauerstoff reagieren und damit durch eine unerwünschte Oxidation an der Doppelbindung spezifische Abbauprodukte bilden (Degradation), die hochgradig unerwünscht sind. Soll weiterhin das Lösungsmittel komplett abgetrennt werden erfordert dies im Allgemeinen eine Hitzebehandlung bei hoher Temperatur, wobei diese Bedingungen der Degradation weiter förderlich sind. Bei Verwendung organischer Lösungsmittel besteht immer Explosionsgefahr, die teure Sicherheitsvorkehrungen nötig macht. Hexan ist in der Diskussion als Nervengift. Es gibt daher gesetzliche Grenzwerte die kontrolliert werden müssen, was die Kosten weiter erhöht. Auch ist die Entsorgung der entsprechenden Abfälle kostenintensiv.

Außerdem kann das Lösungsmittel wie beispielsweise Hexan auch andere Bestandteile aus der Biomasse lösen, die keine Triglyceride sind, und daher Verunreinigungen darstellen, die später entweder gar nicht abgetrennt werden können oder nur mit hohem Aufwand.

Das nach der Abtrennung des Lösungsmittels erhaltene Rohöl muss dann also weiter raffiniert werden, wenn das Öl als Speiseöl und/oder für pharmazeutische Zwecke erhalten werden soll. Die Raffinierungsschritte umfassen das Degummieren (Entschleimung), die Neutralisierung mit alkalischer Lösung, die Entfärbung, das Entwachsen und die Deodorisierung, um die Verunreinigungen wenigstens zu einem Teil zu entfernen. Dies bedeutet jedoch, dass das Öl, welches die hoch ungesättigten Fettsäuren enthält, Bedingungen ausgesetzt wird, bei denen das Auftreten von physikochemikalischen Reaktionen gerade an den ungesättigten Fettsäuren wahrscheinlich wird.

Es gibt jedoch auch Verfahren zur Extraktion von Biomassen, bei denen ein Lösungsmittel nicht verwendet wird, siehe z.B. die EP-A-1178118. Nach dem dort beschriebenen Verfahren wird ein Lösungsmittel vermieden, in dem eine wässrige Suspension der Biomasse hergestellt wird, und die Ölphase durch Zentrifugation aus der wässrigen Phase abgetrennt wird. Die wässrige Phase enthält Zellwandreste und eine bestimmte Menge von wasserlöslichen Material, welches aus der Biomasse stammt. Ein großer Nachteil dieses Verfahrens ist die Tatsache, dass das Rohöl eine Vielzahl von Verunreinigungen enthält wie beispielweise polare Lipide, Proteinreste und so weiter. Das so erhaltene Rohöl muss also über konventionelle Verfahren raffiniert werden wie sie für Pflanzenöle und mikrobielle Öle vorbeschrieben sind.

In der W02004/022678 wird ein Verfahren zur Herstellung PUFA-enthaltender mikrobieller Öle beschrieben, bei dem ein PUFA enthaltendes Öl aus einer Biomasse aus Mikroorganismen durch Pressen erhalten wird. Der verbliebene Presskuchen wird mit einem Trägeröl gemischt, und erneut gepresst, wobei das Trägeröl dabei helfen soll, verbleibende PUFAs aus dem Presskuchen zu lösen, wobei man ein zweites Pressöl erhält, welches eine geringere PUFA-Konzentration aufweist als das erste Pressöl. Durch Vermischen beider Öle in wechselnden Gewichtsanteilen können PUFA-Konzentrationen erhalten werden, die für die jeweils gewünschte Anwendung angepasst sind. Im Prinzip wird hier also ein zweites Öl als Lösungsmittel vorwendet. Dieses Verfahren besitzt im Prinzip die gleichen Nachteile wie sie für den davor beschrieben Stand der Technik zutreffen.

Insgesamt ist das Verpressen von bestimmten Biomassen, insbesondere aus Mikroorganismen wie z.B. *Ulkenia spec.* schwierig und erfordert oft drastische Bedingungen wie hohen Drücken und hohen Temperaturen. Dies geht zu Lasten der Ausbeute und Qualität. Weiterhin entsteht hierdurch eine erhebliche Geruchsbelästigung, welche auch eine Gefährdung des Personals in der Produktionsanlage darstellt.

Außerdem kann das Trägeröl selbst oxidiert werden, worunter die Qualität leiden würde. Im schlimmsten Fall kann sich das Trägeröl durch Oxidation selbst entzünden (bekanntes Problem wenn PUFAs auf großen Oberflächen längere Zeit an der Luft lagern (z.B. Aktivkohle, Bleicherde)).

US 6 255 505 offenbart ein mikrobielles Öl und davon abgeleitete Erzeugnisse, wenigstens umfassend eine PUFA, mit einem Triacylglyceridgehalt von wenigstens 93%. Die Verwendung einer zweiten Biomasse wird in diesem Zusammenhang nicht erwähnt.

WO 97/37032 offenbart ebenfalls ein Verfahren zur Herstellung eines mikrobiel-len Öls und davon abgeleiteter Erzeugnisse. Die Verwendung einer zweiten Biomasse ist auch dieser Druckschrift nicht zu entnehmen.

WO 02/072742 A1 offenbart Öle und phospholipidreiche Zusammensetzungen zur Verwendung als Nahrungsmittel, Arzneimittel und Kosmetika. Die Zusammensetzungen enthalten eine Mischung von mikrobiellen Ölen und Sommenblumenöl.

Wo 92/12711 A1 beschreibt Öle und phospholipidreiche Zusammensetzungen zur Verwendung als Nahrungsmittel, Arzneimittel und Kosmetika. Die Zusammensetzungen enthalten eine Mischung von mikrobiellen DHA-Ölen und Borrage oder Nachtkerzenöl.

Es ist daher Aufgabe der vorliegenden Erfindung, die Nachteile des Standes der Technik zu vermeiden, in dem ein Verfahren zur Herstellung eines stabilen Öls bereitgestellt wird, wobei das Öl eine oder mehrere mehrfach ungesättigten Fettsäuren enthält, welche aus der Biomasse stammt und in Form von Triacylglyceriden oder Phospholipiden in relativ reinem Zustand und hoher Ausbeute vorliegt, und bei.dem das Öl nur einer minimalen Degradation unterliegt.

Soll das ÖL als Speiseöl verwendet werden, ist ein hoher Anteil Triglyceride bevorzugt.

Diese sowie weitere Aufgaben, die sich aus den Nachteilen des diskutierten Stands der Technik für den Fachmann ohne weiteres ergeben, werden gelöst durch ein Verfahren mit allen Merkmalen des Anspruches 1.

Bevorzugte Ausführungsbeispiele der vorliegenden Erfindung sind in den abhängigen unteransprüchen und dem nebengeordneten Ansprüch beschrieben und beansprucht.

Die vorliegende Erfindung betrifft ein Verfahren zur Herstellung eines Öls mit einem Gehalt an mehrfach ungesättigten Fettsäuren ausgewählt aus der Gruppe bestehend aus ARA, DPA, DHA, EPA, GLA, SA oder DHGLA, wobei
a) Mikroorganismen kultiviert werden,
b) die Biomasse aus a) geerntet wird,
c) die erhaltene Biomasse mit einer zweiten, von a) unterschiedlichen Biomasse vermischt wird, und
d) das Öl durch Verpressen der beiden Biomassen gewonnen wird.
wobei die zweite Biomasse der Stufe c) eine Ölsaat ist, ausgewählt aus der Gruppe bestehend aus Sonnenblumen, Raps, Borrage, Nachtkerzen, Leinsamen, Schwarzkümmel, Soja, Palmkern, Palm, Perilla, Saflor, Weizenkeim, Mais, Oliven, Palmkern, Sesam.

Weiterhin betrifft die vorliegende Erfindung einen Presskuchen, erhältlich aus Stufe d) des obigen Verfahrens.

Für die Zwecke der vorliegenden Erfindung wird unter einem Öl auch ein Lipid oder ein Fett verstanden.

Aus dem erhaltenen Rohöl können durch dem Fachmann ohne weiteres bekannte Verfahrensschritte einzelne Bestandteile wie Triglyceride oder Phospholipide aufgereinigt werden, je nach gewünschter Anwendung.

Unter einem bevorzugten Ausführungsbeispiel der vorliegenden Erfindung stellt die zweite Biomasse Sonnenblumensaat dar.

Die zweite Biomasse kann jedoch selbst auch eine mikrobielle Biomasse sein.

Unter einem weiteren bevorzugten Gesichtspunkt wird die mikrobielle Biomasse vor dem Zumischen der zweiten Biomasse getrocknet. Unter einem weiteren bevorzugten Gesichtspunkt stammen die mikrobiellen Biomassen aus der Kultivierung folgender Organismen *Mortierella, Crypthecodinium (Dinoflagellaten), Thraustochytrium, Schizochytrium (Labyrinthulomycetes), Phaeodactylum, Nanochloropsis, Euglena, Tetrahymena, Spirulina* sowie bevorzugt *Ulkenia spec.*

Es hat sich gezeigt, dass bei der Verwendung von Sonnenblumensaat der Pressvorgang erheblich vereinfacht wird. Dies liegt zum Einen daran, dass die Sonnenblumenkerne als zusätzliche Reibungsmittel dienen, die den Aufschluss der Biomasse erleichtern. Außerdem sind Sonnenblumenkerne selbst reich an hochwertigem Pflanzenöl. Dieses wird beim Pressvorgang freigesetzt und dient zum Einen als Schmierstoff für die Mühle, erleichtert so das Verpressen, und das mit freigesetzte Sonneblumenöl dient gleichzeitig als Lösungsmittel für die hohen Anteile an mehrfach ungesättigten Fettsäuren, die als Triglyceride in der mikrobiellen Biomasse vorhanden sind.

Vorteilhaft können Antioxidantien in der Saat/Biomasse einen zusätzlichen stabilisierenden Einfluss auf das Produkt haben. So bildet beispielsweise Crypthecodinium Antioxidantien, die DHA Öl stark stabilisieren können. So bietet das Verfahren auf völlig überraschende Weise einen einfachen Ansatz, mikrobielle Öle, die reich an mehrfach ungesättigten Fettsäuren sind, auf schonende Weise und in hervorragender Ausbeute zur Verfügung zu stellen.

Gleichzeitig kann durch Einstellen der Menge an zugegebenen Sonnenblumenkernen ein Öl erhalten werden, welches unterschiedliche Konzentrationen an PUFAs enthält, und gleichzeitig auch unterschiedliche Mengen an Sonnenblumenölanteilen enthält. Damit können Designeröle entwickelt werden, die spezifisch auf besondere Applikationen angepasst sind.

Auch kann beispielsweise als zweite Biomasse z.B. der Pilz Mortierella eingesetzt werden, der hohe Anteile an ARA (Arachidonsäure) enthält. Bei z.B. Verwendung von Ulkenia als erster Biomasse erhält man ein Öl, welches DHA und ARA enthält. Weiterhin können die Anteile variiert werden, indem der relative Anteil der Biomassen zueinander variiert wird. Wird als weitere Biomasse Sonnenblumensaat oder eine andere Ölpflanze eingesetzt, so wirken sich die in den Pflanzenölen enthaltenen Anti-Oxidantien günstig auf die Lagerstabilität der PUFA-enthaltenden Öle aus.

Die Öle stammen erfindungsgemäß also aus einer oder mehreren mikrobiellen Biomassen ,die vor dem Verpressen optimalerweise mit Ölfrüchten, bevorzugt Sonnenblumensaat vermischt und/oder vermahlen werden.

Die Herstellung des Öls wird also unter direktem Verpressen des Biomassengemisches durchgeführt. Falls nötig, wird dieses erhaltene Öl dann einer physikalischen Raffinierung unterzogen, um das gewünschte Öl zu erhalten.

Für die Zwecke der vorliegenden Erfindung bedeutet physikalische Raffinierung, dass eine Reduktion der Phopholipide und der freien Fettsäuren resultiert, und es wird darunter eine Entschleimungsbehandlung verstanden ohne Verwendung von Säuren und ohne Neutralisierung. So wurde unter Verwendung einer Biomasse, welche ARA enthält, beobachtet, dass das Öle praktisch vollständig frei von Fettsäuren und Phospholipiden war und daher abhängig von dem gewünschten Grad der Reinheit eine Entschleimungsbehandlung nicht nötig war.

Wurde jedoch eine Biomasse verwendet, welche DHA enthielt, war eine Entschleimungsbehandlung notwendig, insbesondere um Phospholipide zu entfernen.

Die erhaltenen Öle sind z.B. geeignet für die Verwendung in Nahrungsmitteln, insbesondere für die Kinderernährung oder zur Verwendung als Nahrungsergänzungsmittel. Sie kann jedoch auch in kosmetischen oder pharmazeutischen Produkten verwendet werden.

Allerdings ist auch die Biomasse, welche nach.dem Pressen verbleibt, ein Verfahrensprodukt, welches bspw. als Tierfuttermittel insbesondere für Haustiere Verwendung findet.

Es ist dabei von besonderem Vorteil, dass die zweite verwendete Biomasse als zusätzliches Aufschlussmittel für die mikrobielle Biomasse wirkt. Wenn die - was normalerweise der Fall ist - mikrobielle Biomasse für sich allein gepresst wird, stellt dies normalerweise an den Pressvorgang sehr hohe Anforderungen. Es wird bei sehr hohen Drücken und oft auch bei erhöhten Temperaturen gepresst, was wiederum schlecht ist für die Qualität des erhaltenen Rohöls, insbesondere bezogen auf mögliche Oxydationsreaktionen der mehrfach ungesättigten Fettsäuren. Wird als zweite zusätzliche Biomasse Sonnenblumensaat verwendet, so dienen die Schalen der Sonnenblumenkerne als Reibe- und Aufschlussmittel, und es kann bei verringerten Drücken und verringerten Temperaturen gearbeitet werden, was die Qualität des erhaltenen PUFA-Öls erheblich verbessert. Diese Effekte werden schon bei nur geringen Mengen an zugegebener zweiter Biomasse gefunden.

Dabei dient das Öl der Ölsaaten als Lösungsmittel für die PUFA-haltigen mikrobiellen Triglyceride, so dass unter einem bevorzugten Ausführungsbeispiel mit dem erfindungsgemäßen Verfahren auf den Einsatz von Lösungsmitteln zum Herauslösen der Triglyceride verzichtet werden kann.

Erfindungsgemäß ist es besonders bevorzugt, wenn als zweite Biomasse Sonnenblumensaat hinzugegeben wird. Es ist jedoch möglich und für den Fachmann selbstverständlich, dass auch andere zweite Biomassen Verwendung finden können. Hedoch handelt es sich bei der zweiten Biomasse um eine pflanzliche Biomasse, und hier insbesondere bevorzugt um eine Biomasse in Form von Pflanzenfrüchten wie bspw. Oliven, Nüsse, Pflanzenblätter, Pflanzenhalme und Pflanzenstängel sowie Bohnen, Schoten und Ähnliches.

Ganz besonders bevorzugt handelt es sich um die klassischen Ölfrüchte, eine Aufstellung der klassischen Ölfrüchte findet sich in den dem Fachmann geläufigen Lehrbüchern und braucht an dieser Stelle nicht wiederholt zu werden. Wie gesagt ist Sonnenblumensaat besonders bevorzugt. Das erfindungsgemäß erhaltene Öl ist besonders gering mit Phospholipiden, freien Fettsäuren, Pigmenten, Polymeren und anderen Substanzen belastet, d.h. Substanzen aus der Biomasse, die keine Triacylglyceride darstellen.

Damit stellt das Verfahren der vorliegenden Erfindung ein besonders selektives Verfahren zur Herstellung von stabilem, hochgereinigte, PUFA enthaltendem Öl dar. Unter einem besonders bevorzugten Gesichtspunkt betrifft vorliegende Erfindung daher ein Verfahren das ohne die aggressiven und hinderlichen Verfahren wie das Entschleimen, die Neutralisierung, das Entwachsen, und die Entfärbung zu einem hochweertigen PUFA-enthaltendem Öl führt.

Allerdings werden die Öle, wo immer es angemessen erscheint, einem Raffinationsschritt unterzogen unter Verwendung eines Prozessierungsmittels wie bspw. einem Silikat. Die Behandlung mit dem Prozessierungsmittel kann bspw. während einer Filtration stattfinden. Schließlich werden die Öle einer Deodorisierung bspw. durch Dampfdestillation oder durch molekulare Destillation bei relativ geringer Temperatur unterzogen. Daraus resultierend enthält das resultierende Öl einen besonders kleinen Anteil von Transfettsäuren.

Das erfindungsgemäße Verfahren umfasst auch die nach der Verpressung angeschlossene Verwendung von organischen Lösungsmitteln für normale Prozesse, wie sie auch beim Verarbeiten von Pflanzenölen anfallen, z.B. dem Winterisieren, Raffinieren, Bleichen, Deodorieren usw. Aloe diese Verfahren sind dem Fachmann geläufig und brauchen für die Zwecke der vorliegenden Erfindung hier nicht erläutert zu werden.

Da das Verfahren bevorzugt unter einer Stickstoffschicht und in Gegenwart von Tokopherolen oder Tokotrienolen durchgeführt wird, die entweder natürlicherweise in der zweiten Biomasse vorkommen, oder während des Verfahrens zugefügt werden, sind die enthaltenen PUFAs während des gesamten Prozesses vor dem schädlichen Einfluss des Luftsauerstoffes geschützt.

Die Verwendung der Biomasse als Nahrungsergänzungsmittel, insbesondere für die Tierzucht ist - gegebenenfalls nach Entfernen der Lösungsmittel - uneingeschränkt möglich.

Es hat sich herausgestellt, dass es besonders günstig ist, wenn die beiden Biomassen vor dem Auspressen mit einander vermahlen werden. Dies ist insbesondere dann von Bedeutung, wenn die zweite Biomasse eine Ölsaat ist, die relativ große Früchte umfasst. Bei der bevorzugten Verwendung von Sonnenblumenkernen als zweite Biomasse hat sich gezeigt, dass ein Vermahlen der Biomassen bis zu einem Grad, bei dem die verbleibende Partikelgröße kleiner als 250 *µ*m, besonders günstig war. Bei diesem Vermahlen wird natürlich bereits ein Teil der enthaltenen Triglyceride freigesetzt in Form von Öl. Für das Vermahlen ist es bspw. möglich, eine Kugelmühle oder eine Koluntmühle zu verwenden. Hierbei ist insbesondere die Dauer des Vermahlens, die Größe der Biomassenpartikel, die Temperatur während des Vermahlens, und das Verhältnis zwischen den beiden Biomassen von Bedeutung. Es ist klar, dass, da ja die verwendete zweite Biomasse in Form von Ölsaaten, insbesondere Sonnenblumenkernen, harte Bestandteile aufweist, diese beim Vermahlen bereits die Biomasse sozusagen beschädigen, und das Öl aus der Biomasse z.T. freigesetzt wird. Dies ist ein besonders günstiger Effekt, der ein besonders schonendes Lösen und gleichzeitig ein Lösen mit Vorausbeute ermöglicht. Deswegen ist dieser Vermahlungsschritt auch ein besonders bevorzugtes Ausführungsbeispiel der vorliegenden Erfindung. Es ist dabei bevorzugt, wenn die resultierenden Partikel der zweiten Biomasse kleiner 500 *µ*m im Durchschnitt aufweisen, bevorzugt kleiner 300 *µ*m und ganz besonders bevorzugt kleiner 200 *µ*m. Diese Größenangaben beziehen sich auf mindestens 90% der anfänglich vorhandenen Biomasse.

Dabei wird der Mahlvorgang solange durchgeführt, bis 90% der eingesetzten Biomasse die gewünschte Partikelgröße aufweist.

Die Temperatur während des Vermahlens wird so gewählt, dass sie oberhalb des Schmelzpunktes des Öls liegt, welche aus der zweiten Biomasse normalerweise gewonnen wird. Ist bspw. die zweite Biomasse Sonnenblumensaat, so wird eine Temperatur gewählt, die oberhalb des Schmelzpunktes von Sonnenblumenöl liegt. Die Temperatur liegt vorzugsweise bei 20-80°C.

Das Gewichtsverhältnis zwischen der mikrobiellen Biomasse und der zweiten Biomasse liegt zwischen 100:1 bis 1:100. Wie weiter oben ausgeführt, bestimmt ja das Verhältnis aus beiden Biomassen ganz wesentlich die PUFA-Konzentration im erhaltenen Öl. Dabei sind sicherlich Verhältnisse bei denen ein PUFA-Gehalt von zwischen 1 und 10% im fertigen Designeröl erzielt wird, bevorzugt, da es sich hierbei um Konzentrationen handelt, wie sie bei den meisten pharmazeutischen, kosmetischen und nahrungsmitteltechnischen Anwendungen von Vorteil sind. Wird bspw. eine Biomasse erhalten, bei der über 40% PUFAs durch Auspressen der Biomasse erhalten werden können, so kann ein bevorzugtes Verhältnis zwischen mikrobieller Biomasse und zweiter Biomasse, insbesondere der Ölfrüchte, so gewählt sein, dass das resultierende Öl etwa 10% PUFA umfasst. Dazu muss die normale Ausbeute an Öl der zweiten Biomasse in Betracht gezogen werden, welche jedoch dem Fachmann bei den herkömmlichen Ölfrüchten jederzeit ohne größere Probleme bekannt ist.

Das erhaltene Öl muss dann noch u.U. einer Feinfiltration unterzogen werden, um kleine unlösliche Partikel zu entfernen. Hierzu sind dem Fachmann viele unterschiedliche Verfahren bekannt, bspw. kann das Öl einem mineralischen Adsorbents als Filterhilfsmittel ausgesetzt werden, bspw. Kieselerdefiltration.

Schlussendlich wird das gefilterte Öl gärungsfrei gemacht, wobei flüchtige Substanzen entfernt werden. Dies kann ebenfalls unter Verwendung der im Stand der Technik bekannten Verfahren durchgeführt werden, es sollten jedoch moderate Bedingungen verwendet werden, um den PUFAs keinen Schaden zuzufügen. Bspw. kann die Dampfdestillation, vorzugsweise unter Vakuum oder die molekulare Destillation Verwendung finden.

Das so erhaltene Öl kann bspw. in Nahrungsmittelzusammensetzungen für die menschliche Ernährung verwendet werden, so wie es ist, oder in Form eines Gemisches mit anderen Ölen wie bspw. Fischölen oder Salatölen oder alternativ in Form einer Emulsion als Salatdressing oder Mayonnaise. Es kann Bestandteil einer diätetischen Milch für Teenager oder Erwachsene sein, als Babynahrung für ungestillte Babys oder als Milch für kleine Kinder dienen. Es kann ebenfalls in eine pharmazeutische Zusammensetzung für die orale, enterale oder parenterale Verabreichung eingebaut werden oder für die topische, dermatologische oder ophthalmologische Anwendung. Es kann ferner ein Bestandteil einer kosmetischen, topischen oder oralen Zusammensetzung sein. Schlussendlich kann es auch als Tiernahrungsmittel dienen, bspw. als Trocken- oder Feuchtfutter oder als Milch. Die verbleibenden Biomasse kann immer noch mit Vorteil als Tierfutter und für die obigen Zwecke eingesetzt werden.

Außerdem kann aus der verbleibenden Biomasse mit Vorteil unter Verwendung einer Extraktion mit einem Lösungsmittel, bevorzugt einem organischen Lösungsmittel und besonders bevorzugt mit einem unpolaren, mit Wasser nicht mischbarem Lösungsmittel, wobei Hexan besonders bevorzugt ist, extrahiert werden, um den durch die Pressung nicht herausgelösten Teils an Öl gewinnen zu können. Die Hexanextraktion solcher Biomassen ist im Stand der Technik ausführlich beschreiben, eine umfassende Darstellung findet sich beispielsweise in der EP 0 515 460 der Martek Corp.

Es sind auf dem Fachgebiet neben der Hexanextraktion noch eine Reihe weiterer Extraktionsverfahren bestens bekannt, die an dieser Stelle geeignet sind, das Öl, bevorzugt mit hohem Triglyceridgehalt nach dem Abpressen aus dem verbleibenden Presskuchen zu gewinnen, z.B. die Extraktion mit Chloroform/ Methanol oder auch mit superkritischem CO₂.

## Patentansprüche

1. Verfahren zur Herstellung eines Öls mit einem Gehalt an mehrfach ungesättigten Fettsäuren ausgewählt aus der Gruppe bestehend aus ARA, DPA, DHA, EPA, GLA, SA oder DHGLA, wobei
a) Mikroorganismen kultiviert werden,
b) die Biomasse aus a) geerntet wird,
c) die erhaltene Biomasse mit einer zweiten, von a) unterschiedlichen Biomasse vermischt wird, und
d) das Öl durch Verpressen der beiden Biomassen gewonnen wird.
**dadurch gekennzeichnet, dass** die zweite Biomasse der Stufe c) eine Ölsaat ist, ausgewählt aus der Gruppe bestehend aus Sonnenblumen, Raps, Borrage, Nachtkerzen, Leinsamen, Schwarzkümmel, Soja, Palmkern, Palm, Perilla, Saflor, Weizenkeim, Mais, Oliven, Palmkern, Sesam.

2. Verfahren nach Anspruch 1, wobei nach Stufe d) der verbleibende Presskuchen einer Extraktion mit einem Lösungsmittel für das Öl, bevorzugt einem organischen Lösungsmittel und besonders bevorzugt mit einem unpolaren, mit Wasser nicht mischbarem Lösungsmittel, wobei Hexan besonders bevorzugt ist, unterzogen wird, und das Öl nach Extraktion von dem Lösungsmittel gewonnen wird.

3. Verfahren nach Anspruch 1, wobei das gewonnene Öl aus Stufe d) mit einem nach dem Verfharen nach Anspruch 2 gewonnenen Öl vermischt wird.

4. Verfahren nach einem der vorstehenden Ansprüche ,
**dadurch gekennzeichnet, dass** das Verfahren bei einer Temperatur durchgeführt wird, die mindestens 5°C, bevorzugt 10° C oberhalb des Schmelzpunktes des am niedrig schmelzenden aus den beiden Biomassen erhältlichen Öle, insbesondere des aus der zweiten Biomasse erhältlichen Öls, und ganz besonders bevorzugt zwischen 20 und 80°C liegt.

5. Verfahren nach einem der vorstehenden Ansprüche, **dadurch gekennzeichnet, dass** nach Stufe d) das Öl schonend lösungsmittelfrei und ohne Verwendung basischer oder saurer Mittel raffiniert wird, indem mindestens eine der folgenden Stufen durchgeführt wird:
a) Prozessierung unter Verwendung von Silikat, und/oder
b) Prozessierung unter Verwendung von Kieselerdefiltration, und/oder
c) Deodorierung unter Dampfdestillation.

6. Verfahren nach einem der vorstehenden Ansprüche, **dadurch gekennzeichnet, dass** die erste Biomasse mindestens eine Biomasse aus einer der folgenden Mikroorganismen umfasst *Mortierella, Crypthecodinium (Dinoflagellaten), Thraustochytrium, Schizochytrium (Labyrinthulomycetes), Phaeodactylum, Nanochloropsis, Euglena, Tetrahymena, Spirulina, Chlorella* und *Ulkenia,* wobei *Ulkenia* bevorzugt ist.

7. Verfahren nach einem der vorstehenden Ansprüche, **dadurch gekennzeichnet, dass** die beiden Biomassen vor Beginn des Pressvorgangs miteinander vermahlen werden.

8. Verfahren nach Anspruch 7, **dadurch gekennzeichnet, dass** das Vermahlen bei einer Temperatur durchgeführt wird, die mindestens 5°C, bevorzugt 10° C oberhalb des Schmelzpunktes des am niedrig schmelzenden aus den beiden Biomassen erhältlichen Öle, insbesondere des aus der zweiten Biomasse erhältlichen Öls, und ganz besonders bevorzugt zwischen 20 und 80°C liegt.

9. Presskuchen, erhältlich aus Stufe d) aus Anspruch 1.

## Claims

1. A method for the manufacture of an oil containing polyunsaturated fatty acids selected from the group formed by ARA, DPA, DHA, EPA, GLA, SA and DHGLA, wherein
a) microorganisms are cultivated,
b) the biomass from a) is harvested,
c) the biomass obtained is mixed with a second biomass which is different from a), and
d) the oil is obtained by pressing the two biomasses,
**characterized in that** the second biomass of step c) is an oil seed selected from the group consisting of sunflower seed, rapeseed, borage, evening primrose, linseed, black cumin, soya, palm kernel, palm, perilla, safflower, wheatgerm, corn, olive, palm kernel and sesame seed.

2. The method as claimed in claim 1, wherein after step d), the remaining press cake undergoes an extraction with a solvent for the oil, preferably with an organic solvent and particularly preferably with a non-polar solvent which is not miscible with water, wherein hexane is particularly preferred and the oil is obtained after extraction from the solvent.

3. The method as claimed in claim 1, wherein the oil obtained from step d) is mixed with an oil obtained in accordance with the method as claimed in claim 2.

4. The method as claimed in one of the preceding claims, **characterized in that** the method is carried out at a temperature which is at least 5°C, preferably 10°C above the melting point of the oils which have the lowest melting point obtainable from the two biomasses, in particular the oil obtainable from the second biomass, and particularly preferably between 20°C and 80°C.

5. The method as claimed in one of the preceding claims, **characterized in that** after step d), the oil is gently refined without a solvent and without using basic or acidic substances, wherein at least one of the following steps is carried out:
a) processing using silicate, and/or
b) processing by silica filtration, and/or
c) deodorizing using steam stripping.

6. The method as claimed in one of the preceding claims, **characterized in that** the first biomass comprises at least one biomass from one of the following microorganisms: *Mortierella, Crypthecodinium (Dinoflagellates), Thraustochytrium, Schizochytrium (Labyrinthulomycetes), Phaeodactylum, Nanochloropsis, Euglena, Tetrahymena, Spirulina, Chlorella* and *Ulkenia,* wherein *Ulkenia* is preferred.

7. The method as claimed in one of the preceding claims, **characterized in that** prior to commencing the pressing procedure, the two biomasses are ground together.

8. The method as claimed in claim 7, **characterized in that** grinding is carried out at a temperature which is at least 5°C, preferably 10°C above the melting point of the oils which have the lowest melting point obtainable from the two biomasses, in particular the oil obtainable from the second biomass, and par-ticularly preferably between 20°C and 80°C.

9. A press cake which can be obtained from step d) of claim 1.

## Revendications

1. Procédé de fabrication d'une huile composée d'acides gras polyin-saturés choisis dans le groupe constitué d'ARA, DHA, EPA, GLA, SA ou DHGLA, au cours duquel
a) des micro-organismes sont cultivés
b) la biomasse obtenue en a) est récoltée,
c) cette biomasse est mélangée avec une deuxième biomasse différente de celle en a), et
d) l'huile est obtenue par le pressage des deux biomasses.
**caractérisé en ce que** la deuxième biomasse de l'étape c) est un oléagineux choisi dans le groupe composé de tournesols, colzas, bouraches, onagres, graines de lin, nigelle cultivée, soja, graines de palmier, palmiers, perillas, carthames, germes de blé, maïs, olives, graines de palmier, sésame.

2. Procédé selon la revendication 1, au cours duquel les tourteaux issus de l'étape d) sont soumis à un procédé d'extraction avec un solvant pour l'huile, de préférence un solvant biologique et au mieux avec un solvant apolaire et non miscible à l'eau, l'hexane étant privilégié, et l'huile est obtenue après extraction par le sol-vant.

3. Procédé selon la revendication 1, au cours duquel l'huile obtenue en d) est mélangée avec l'huile obtenue avec le procédé selon la revendication 2.

4. Procédé selon l'une des revendications précédentes, **caractérisé en ce que** le procédé est effectué à une température supérieure d'au moins 5 °C et préférablement 10 °C au point de fusion de l'huile au point de fusion le plus bas obtenue à partir des deux biomasses, plus particulièrement de l'huile obtenue à partir de la deuxième biomasse, comprise au mieux entre 20 et 80 °C.

5. Procédé selon l'une des revendications précédentes, **caractérisé en ce que** l'huile obtenue selon l'étape d) est soigneusement raffinée sans solvant et sans l'utilisation d'agents basiques ou acides au moyen d'au moins l'une des étapes suivantes :
a) Transformation à l'aide de silicate, ou
b) transformation par filtration de silice, ou
c) désodorisation par distillation à la vapeur.

6. Procédé selon l'une des revendications précédentes, **caractérisé en ce que** la première biomasse comprend au moins une biomasse composée de l'un des micro-organismes suivants : *mortierella, crypthecodinium (dinophytes), thraustochytrium, achizochytrium (labyrinthulomycetes), phaeodactylum, nanochloropsis, euglena, tetrahymena, spirulina, chlorella* et *ulkenia,* l'*ulkenia* étant privilégiée.

7. Procédé selon l'une des revendications précédentes, **caractérisé en ce que** les deux biomasses sont broyées ensemble avant que ne soit commencé le pressage.

8. Procédé selon la revendication 7, **caractérisé en ce que** le procédé est effectué à une température supérieure d'au moins 5 °C et préférablement 10 °C au point de fusion de l'huile au point de fusion le plus bas obtenue à partir des deux biomasses, plus particulièrement de l'huile obtenue à partir de la deuxième biomasse, comprise au mieux entre 20 et 80 °C.

9. Tourteaux obtenus selon l'étape d) de la revendication 1.
